# EUROPEAN PATENT APPLICATION

(11) **EP 3 363 489 A1**
(43) Date of publication of application: **22.08.2018**
(21) Application number: 16855300.6
(22) Date of filing: 04.10.2016
(51) Int. Cl.: A61M 16/16, F24F 3/04, F24F 6/08

(54) **HUMIDIFIER AND RESPIRATORY ASSIST DEVICE**

(30) Priority: 16.10.2015 JP 2015205051
(71) Applicant: Metran Co., Ltd., Kawaguchi-shi, Saitama 332-0015 (JP)
(72) Inventor: NITTA, Kazufuku, Kawaguchi-shi Saitama 332-0015 (JP)
(74) Representative: TBK
(86) International application number: PCT/JP2016/079389
(87) International publication number: WO 2017/065046

(57) **Abstract**

A respiratory assistance device includes a gas supply source, an inspiratory pipe, and a humidifier. A gas is supplied to a mask through the gas supply source and the inspiratory pipe. The humidifier includes a liquid supply unit, a humidifying member, a holder unit, a heating unit, and the like. The liquid supply unit supplies a liquid to the humidifying member. The humidifying member has an aspect that makes one round of the inner periphery of the inspiratory pipe, and vaporizes the liquid supplied from the liquid supply unit. The holder unit holds the humidifying member such that the humidifying member is disposed to make one round of the inner periphery of the inspiratory pipe in a continuous or discontinuous manner. The heating unit heats the inside of the inspiratory pipe. This can provide a humidifier having an improved humidifying performance and a respiratory assistance device that have simple structures and ease of use.

## Description

### Technical Field

The present invention relates to a humidifier and a respiratory assistance device.

### Background Art

Respiratory assistance devices such as artificial respirators are used by patients having respiratory disturbance such as sleep apnea syndrome (SAS). The respiratory assistance device feeds a gas, such as compressed air, supplied from a blower and the like into an airway of the patient through an inspiratory pipe, after humidifying the gas with a humidifier. Such respiratory assistance devices having the humidifiers are used not only in medical facilities but also in homes. Therefore, a respiratory assistance device having a simple structure and ease of use is required. In such circumstances, the applicant invented the following respiratory assistance device (refer to, for example, Japanese Patent Application No. 2014-016734).

The respiratory assistance device is provided with the following humidifier. The humidifier includes a container configured to contain a liquid, a feeding mechanism configured to feed the liquid from the container by reducing the capacity of the container with time, and a water permeable member that is disposed in an inspiratory pipe configured to lead air into an airway of a user of the respiratory assistance device, the water permeable member being configured to vaporize the liquid supplied from the container.

Owing to its simple structure and ease of use, this respiratory assistance device is convenient for a user who uses the respiratory assistance device in his or her home.

### Summary of Invention

### Technical Problem

To make the respiratory assistance device more convenient, the applicant has continued development of the respiratory assistance device. As a result, the applicant has judged that its humidifying performance can be more improved.

Considering the aforementioned circumstances, the present invention aims at providing a humidifier having an improved humidifying performance and a respiratory assistance device that have simple structures and ease of use.

### Solution to Problem

The present invention has been made to solve the above object, and a humidifier according to the present invention includes: an inspiratory pipe; and a humidifying member configured to hold and vaporize a liquid. The humidifying member is disposed in the shape of a pipe along an inner periphery of the inspiratory pipe in a continuous or discontinuous manner in a circumferential direction, so that intake air passes through the pipe, or is disposed so as to partition an interior space of the inspiratory pipe, so that intake air passes through the partitioned space. The present invention thereby provides the effect of uniformly humidifying the gas inside the inspiratory pipe.

In the humidifier according to the present invention, the humidifying member has an aspect in which the humidifying member makes at least one round of the inner periphery of the inspiratory pipe.

In the humidifier according to the present invention, the humidifying member has an aspect in which a plurality of plane members are disposed radially.

In the humidifier according to the present invention, the humidifying member has an aspect in which a plurality of plane members are disposed into a lattice shape.

In the humidifier according to the present invention, the humidifying member is formed from at least any one material out of woven cloth, nonwoven cloth, and a water-absorbing polymer.

In the humidifier according to the present invention, the humidifying member is formed from a plurality of hollow fibers.

The humidifier according to the present invention includes a liquid supply unit configured to supply the liquid; and a leading unit configured to lead the supplied liquid to end portion openings of the plurality of hollow fibers.

In the humidifier according to the present invention, the liquid supply unit includes: a pump configured to circulate the liquid in a liquid circulation system to supply the liquid to the plurality of hollow fibers; a leading unit configured to lead the liquid so as to form a reciprocating tube together with the plurality of hollow fibers; and a supply tube and a return tube that are connected to the leading unit corresponding to an inlet and an outlet of the reciprocating tube, respectively, to form the liquid circulation system together with the plurality of hollow fibers.

In the humidifier according to the present invention, the reciprocating tube is formed from a first tube formed from a part of the plurality of hollow fibers, and a second tube formed from a remaining part of the hollow fibers. The leading unit is formed from a first leading member configured to connect the supply tube to an end portion opening of the first tube, a second leading member configured to connect the return tube to an end portion opening of the second tube, and a third leading member configured to connect the other end portion opening of the first tube to the other end portion opening of the second tube. The liquid circulation system is formed from the supply tube and the return tube, the first tube, the second tube, the first leading member, the second leading member, and the third leading member.

In the humidifier according to the present invention, the reciprocating tube is formed by bending the plurality of hollow fibers into a reciprocating aspect. The leading unit is formed from a first leading member configured to connect the supply tube to one end portion openings of the plurality of hollow fibers, and a second leading member configured to connect the return tube to other end portion openings of the plurality of hollow fibers. The liquid circulation system is formed from the supply tube and the return tube, the plurality of hollow fibers, the first leading member, and the second leading member.

In the humidifier according to the present invention, the liquid supply unit includes a container configured to contain the liquid; and a feeding mechanism configured to gradually reduce a capacity of the container with time, to feed the liquid from the container and supply the liquid to the humidifying member.

In the humidifier according to the present invention, the liquid supply unit includes a container configured to contain the liquid; and a pressurization unit configured to apply a pressure to an inside of the container.

In the humidifier according to the present invention, the pressurization unit applies a pressure by, when the pressurization unit itself is retracted by an external force, feeding air held inside the pressurization unit itself to the inside of the container.

In the humidifier according to the present invention, when a difference between a pressure of the inside of the container and a pressure of an inside of the inspiratory pipe is equal to or lower than a predetermined threshold value, the pressurization unit applies a pressure to the inside of the container.

In the humidifier according to the present invention, when the pressure of the inside of the container is equal to or lower than a first threshold value, the pressurization unit applies a pressure to the inside of the container.

In the humidifier according to the present invention, when the pressure of the inside of the inspiratory pipe is equal to or more than a second threshold value, the pressurization unit applies a pressure to the inside of the container.

The humidifier according to the present invention includes a holder unit configured to hold the humidifying member so as to dispose the humidifying member in the shape of the pipe along the inner periphery of the inspiratory pipe in a continuous or discontinuous manner in the circumferential direction.

In the humidifier according to the present invention, the holder unit is a helical member.

In the humidifier according to the present invention, the holder unit is a ring continuation member in which ring-shaped members are continuously arranged and connected to each other in an axial direction of the inspiratory pipe.

In the humidifier according to the present invention, a heating unit is provided outside or inside the inspiratory pipe, in order to heat the humidifying member.

In the humidifier according to the present invention, when the humidifying member is disposed in the shape of the pipe along the inner periphery of the inspiratory pipe in a continuous or discontinuous manner in the circumferential direction, the heating unit is provided with a heating unit configured to heat the inside of the inspiratory pipe from an outer peripheral side of the humidifying member, inside or outside the inspiratory pipe.

In the humidifier according to the present invention, the heating unit is disposed helically on an outer periphery of the inspiratory pipe.

In the humidifier according to the present invention, a projection portion is helically formed in the outer periphery of the inspiratory pipe, and a depression portion is helically formed alongside the projection portion, and the heating unit is helically disposed in the depression portion.

In the humidifier according to the present invention, a heating unit is provided outside or inside the inspiratory pipe, in order to heat the inside of the inspiratory pipe from an outer peripheral side of the humidifying member.

In the humidifier according to the present invention, the heating unit is disposed so as to heat the intake air inside the inspiratory pipe.

In the humidifier according to the present invention, the heating unit is disposed so as to heat condensation water accumulated in the inspiratory pipe.

The humidifier according to the present invention includes a container configured to contain the liquid. The humidifying member is disposed in the inspiratory pipe in a state where a part of the humidifying member is inserted in the container, to absorb the liquid from the container and vaporize the liquid.

In the humidifier according to the present invention, the humidifying member is formed from a water-absorbing material that can absorb the liquid in the container.

In the humidifier according to the present invention, the humidifying member is formed from a water-absorbing material that can absorb the liquid in the container by capillarity.

In the humidifier according to the present invention, the humidifying member is formed from a porous material.

In the humidifier according to the present invention, the porous material is formed from at least any one material out of woven cloth, nonwoven cloth, a hollow fiber, a porous water-absorbing polymer, and a porous synthetic resin.

The humidifier according to the present invention includes a heating unit configured to heat the liquid in the container.

A respiratory assistance device according to the present invention includes the humidifier described above. Advantageous Effects of Invention

The present invention can provide a superior effect that the gas can be uniformly humidified in the inspiratory pipe of the respiratory assistance device.

### Brief Description of Drawings

FIG. 1 is a drawing illustrating a respiratory assistance device 1 according to an embodiment of the present invention.
FIG. 2 is a drawing illustrating a humidifying member 17 and a holder unit 18 according to the embodiment of the present invention.
FIG. 3 is a drawing illustrating examples of structures of holder units 118 to 328 according to other embodiments of the present invention.
FIG. 4 is a sectional view illustrating examples of structures of humidifying members 417 to 717 and holder units 418 to 718 according to other embodiments of the present invention.
FIG. 5 is a drawing illustrating aspects of humidifying members 817 and 917 according to other embodiments of the present invention.
FIG. 6 is a drawing illustrating a respiratory assistance device 2 according to another embodiment of the present invention.
FIG. 7 is a drawing illustrating a humidifying member 27 and a holder unit 28 according to another embodiment of the present invention.
FIG. 8 is a drawing illustrating a respiratory assistance device 3 according to another embodiment of the present invention.
FIG. 9 is a drawing illustrating a respiratory assistance device 4 according to another embodiment of the present invention.
FIG. 10 is a drawing illustrating a respiratory assistance device 5 according to another embodiment of the present invention.
FIG. 11 is a drawing illustrating a respiratory assistance device 6 according to another embodiment of the present invention.
FIG. 12 is a drawing illustrating a respiratory assistance device 7 according to another embodiment of the present invention.
FIG. 13 is a drawing illustrating a state of using an accordion pipe 71b, as an inspiratory pipe 71.
FIG. 14 is a drawing illustrating a respiratory assistance device 8 according to another embodiment of the present invention.
FIG. 15 is a drawing showing modification examples of the humidifying member in the respiratory assistance device 8. Description of Embodiments

Embodiments of the present invention will be described below with reference to the accompanying drawings.

### <1. Respiratory assistance device 1>

### <1-1. Whole structure of respiratory assistance device 1>

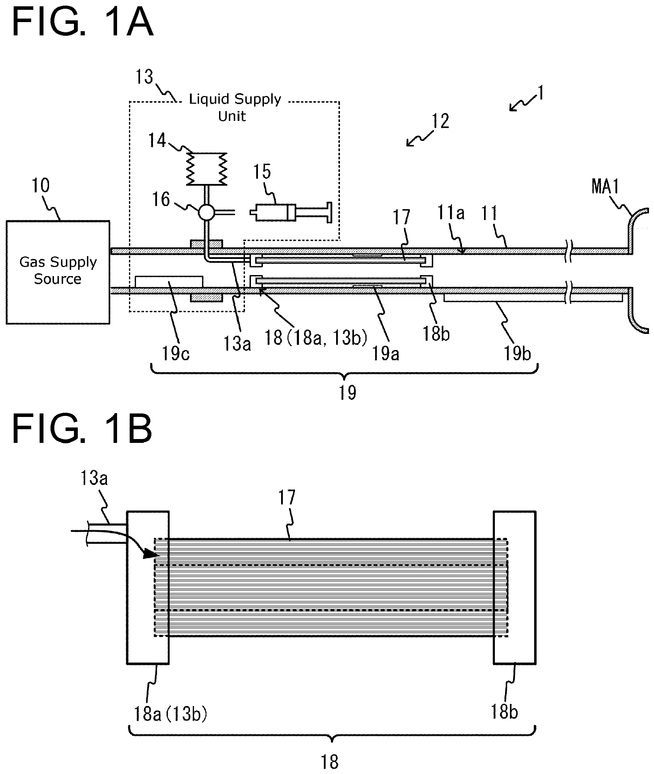

FIG. 1 is a drawing illustrating a respiratory assistance device 1 according to an embodiment of the present invention. FIG. 1A is a sectional view illustrating the whole structure of the respiratory assistance device 1. FIG. 1B is a drawing illustrating a humidifying member 17 and a holder unit 18 in the respiratory assistance device 1.

The respiratory assistance device 1 is used by a patient (user) having respiratory disturbance, to feed a gas (compressed air, oxygen, or the like) into an airway of the patient as an intake air. More specifically, the respiratory assistance device 1 includes a gas supply source 10, an inspiratory pipe 11, a humidifier 12, and the like.

The gas supply source 10 supplies the gas. As the gas supply source 10, a well-known oxygen tank, an air blower, or the like is used. The details of the air blower are described in, for example, Japanese Patent No. 5211302.

An end of the inspiratory pipe 11 is connected to the gas supply source 10. The other end of the inspiratory pipe 11 is connected to, for example, a mask MA1 that covers the mouth and nose of the patient. The inspiratory pipe 11 leads the gas into the airway of the patient. Note that the illustration of an expiratory circuit, including an expiratory valve and the like, is omitted.

The humidifier 12 humidifies the gas inside the inspiratory pipe 11. To be more specific, the humidifier 12 includes, for example, a liquid supply unit 13, a humidifying member 17, a holder unit 18, a heating unit 19, and the like. The liquid supply unit 13 supplies a liquid to the humidifying member 17.

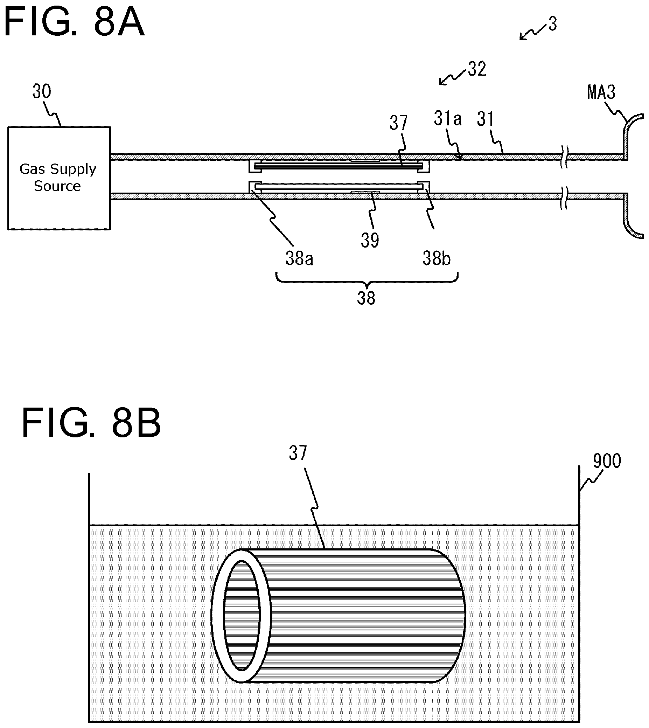

The humidifying member 17 vaporizes the liquid supplied from the liquid supply unit 13. The humidifying member 17 is formed from, for example, water permeable fibers, which are hollow fibers having high water permeability, or the like. The water permeable fiber is a hollow fiber that allows water held inside a hollow to permeate from a side surface of the hollow fiber little by little. The humidifying member 17 may be formed from, for example, gas permeable fibers, which are hollow fibers having gas permeability. The gas permeable fiber is a hollow fiber that, when water held inside a hollow is vaporized, allows the vapor to permeate from a side surface of the hollow fiber. Note that the humidifying member 17 may be formed from nonwoven cloth or a water-absorbing polymer. This will be further described below using FIG. 8. In the following description, it is assumed that the humidifying member 17 is formed from the water permeable fibers.

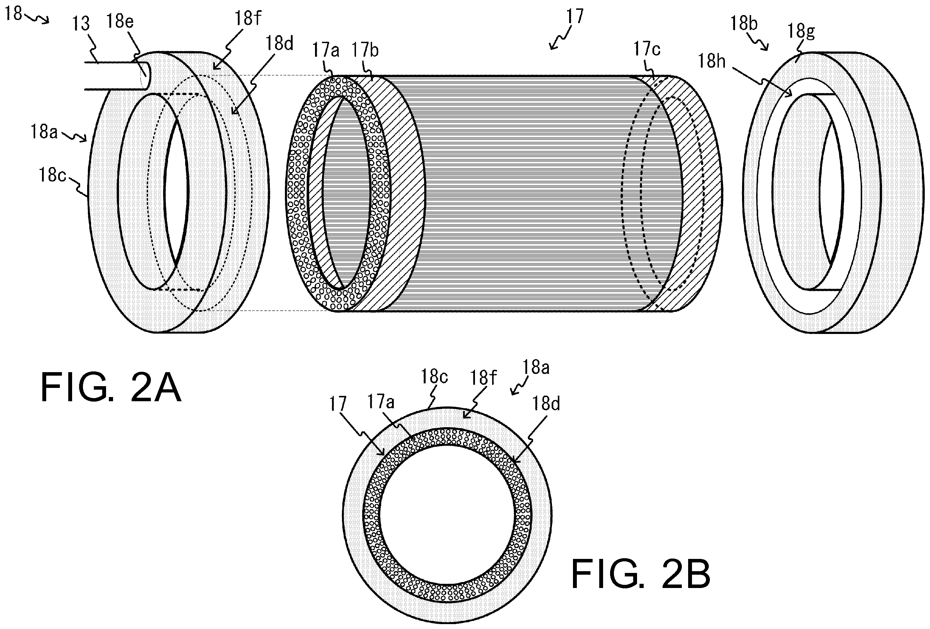

The humidifying member 17 has a pipe-shaped aspect along an inner periphery of the inspiratory pipe 11 in a continuous or discontinuous manner in a circumferential direction. As an example of the humidifying member 17, for example, a bundle of water permeable fibers that extend along a length direction of the inspiratory pipe 11 and is formed into a cylindrical shape as a whole is conceivable, but not limited thereto, and the humidifying member 17 may have another aspect. In FIG. 1, the humidifying member 17 has a cylindrical aspect that continuously makes one round along an inner peripheral surface 11a of the inspiratory pipe 11, in a certain section of the inspiratory pipe 11. Note that the aspect of the humidifying member 17 will be further described later using FIG. 2.

The holder unit 18 holds the humidifying member 17 such that the humidifying member 17 is disposed in the shape of a pipe along the inner periphery of the inspiratory pipe 11 of the respiratory assistance device 1 in a continuous or discontinuous manner in the circumferential direction. In FIGS. 1A and 1B, the holder unit 18 holds the humidifying member 17 such that the humidifying member is disposed to make one round of the inner periphery of the inspiratory pipe. By way of example, as shown in FIGS. 1A and 1B, the holder unit 18 has an aspect that holds both end portions of the humidifying member 17. In this case, the holder unit 18 is constituted by a holder member 18a, a holder member 18b, and the like.

The holder member 18a holds a left end portion of the humidifying member 17. The holder member 18a is connected to a supply tube 13a. As illustrated in FIG. 1B, the liquid supplied from the liquid supply unit 13 through the supply tube 13a is supplied to the humidifying member 17 through the holder member 18a. The liquid penetrates into the humidifying member 17.

The holder member 18b holds a right end portion of the humidifying member 17. The concrete aspects of the holder members 18a and 18b, which constitute the holder unit 18, will be further described later using FIG. 2.

As described above, by disposing the humidifying member 17 inside the inspiratory pipe 11, the gas inside the inspiratory pipe 11 of the respiratory assistance device 1 can be uniformly humidified. The humidifying member 17 is disposed such that the intake air passes through the inside of the humidifying member 17 itself, and therefore has a large humidifying area for the intake air. Therefore, the humidifying member 17 can sufficiently humidify the intake air.

The heating unit 19 heats the inside of the inspiratory pipe 11. The heating unit 19 may be provided either inside or outside of the inspiratory pipe 11. In the respiratory assistance device 1 according to the embodiment of the present invention, the heating unit 19 is constituted by, for example, a heating unit 19a, a heating unit 19b, and a heating unit 19c.

The heating unit 19a is disposed on an outer periphery of the humidifying member 17 inside the inspiratory pipe 11, in order to heat the liquid held by the humidifying member 17 and the intake air inside the inspiratory pipe 11. Note that the heating unit 19a may be disposed in any position either inside or outside the inspiratory pipe 11, as long as the heating unit 19a can heat the liquid and the intake air inside the inspiratory pipe 11. The heating unit 19a may have an aspect that encloses the outer periphery of the humidifying member 17 in a continuous or discontinuous manner. In this case, the heating unit 19a is effectively used for uniformly heating the intake air inside the inspiratory pipe 11. The heating unit 19a is also effectively used for heating and vaporizing the liquid held by the humidifying member 17. The heating unit 19a is also effectively used for heating and vaporizing condensation water that is condensed outside the humidifying member 17.

The heating unit 19b is disposed on an outer periphery of the inspiratory pipe 11 downstream of the inspiratory pipe 11 from the humidifying member 17, in order to heat condensation water accumulated in the inspiratory pipe 11. Note that the heating unit 19b may be disposed in any position either inside or outside the inspiratory pipe 11, as long as the heating unit 19b can heat the condensation water accumulated in the inspiratory pipe 11. The condensation water accumulated in the inspiratory pipe 11 causes breeding of germs and hence hygienic problems. Thus, the condensation water is vaporized by heating by the heating unit 19b. The heating unit 19b is also effectively used for heating the intake air downstream of the inspiratory pipe 11 from the humidifying member 17.

The heating unit 19c is disposed inside the inspiratory pipe 11 upstream of the inspiratory pipe 11 from the humidifying member 17, in order to heat the intake air supplied from the gas supply source 10. Note that the heating unit 19c may be disposed in any position either inside or outside the inspiratory pipe 11, as long as the heating unit 19c can heat the intake air. The heating unit 19c is also effectively used for heating and vaporizing condensation water that is condensed on the inner periphery of the inspiratory pipe 11 upstream of the inspiratory pipe 11 from the humidifying member 17.

Next, an example of the liquid supply unit 13 will be described. The liquid supply unit 13 includes a container 14, an injector 15, a three-way valve 16, a leading unit 13b, and the like.

The container 14 having airtightness is a vessel for containing a required amount (for example, 60 cc for one night (8 hours)) of liquid (water or the like). The container 14 is connected to each of the injector 15 and the humidifying member 17 via the three-way valve 16. Thus, the container 14 is indirectly connected to the humidifying member 17, and indirectly connected to the injector 15.

The container 14 is formed from a stretchable accordion material or the like having stretchability (expandability and retractability). An injection of the liquid by the injector 15 expands the container 14. The container 14 retracts, owing to retractability of itself, and gradually reduces the capacity of itself with time (for example, over one night (8 hours)) to feed the liquid into the humidifying member 17. In other words, the container 14 functions as a feeding mechanism that feeds a liquid contained in itself by reducing the capacity of itself with time.

The humidifying member 17 is indirectly connected to the container 14 so as to be coupled with the container 14 through the three-way valve 16. The humidifying member 17 is supplied with the liquid from the container 14, and naturally vaporizes the liquid.

As the injector 15, a well-known syringe or the like is used. The injector 15 is indirectly and detachably connected to the container 14 so as to be coupled to the container 14 through the three-way valve 16. By operation of the injector 15, the liquid is injected into the container 14 by an amount corresponding to the capacity of the container 14.

The three-way valve 16 connects the container 14, the injector 15, and the humidifying member 17, as described above. The three-way valve 16 allows leading the liquid from the injector 15 to the container 14, while prevents leading the liquid from the injector 15 to the humidifying member 17. The three-way valve 16 allows leading the liquid from the container 14 to the humidifying member 17, while prevents leading the liquid from the container 14 to the injector 15. The three-way valve 16 also prevents leading the liquid from the humidifying member 17 to the container 14 and the injector 15. The three-way valve 16 thereby leads the liquid from the injector 15 to the container 14, and leads the liquid from the container 14 to the humidifying member 17.

The leading unit 13b leads the liquid supplied through the supply tube 13a to one-end openings of all the plurality of water permeable fibers constituting the humidifying member 17. When a lot of water permeable fibers are bundled along the length direction of the inspiratory pipe 11 into the shape of a pipe as a whole, the liquid supplied through the supply tube 13a penetrates into the plurality of water permeable fibers from end openings thereof. As illustrated in FIG. 1B, the holder member 18a leads the liquid supplied through the supply tube 13a to the one-end openings of all the plurality of water permeable fibers. Therefore, in this case, the holder member 18a also functions as the leading unit 13b.

### <1-2. Humidifying member 17 and holder unit 18>

FIG. 2 is a drawing illustrating the humidifying member 17 and the holder unit 18 according to the embodiment of the present invention. FIG. 2A is a perspective view of the humidifying member 17 and the holder unit 18. FIG. 2B is a sectional view when the humidifying member 17 is attached to the holder unit 18.

As illustrated in FIG. 2A, the humidifying member 17 is formed from a plurality of water permeable fibers 17a bundled in the length direction into a cylindrical shape. By way of example, the humidifying member 17 is formed as follows.

First, a hollow pipe-shaped jig having a circular ring-shaped opening is prepared. This jig may have a similar aspect to the holder member 18b illustrated in FIG. 2A. A plurality of water permeable fibers 17a are inserted into the ring opening of the jig. The water permeable fibers 17a are thereby inserted into the jig by a certain length from end portions of the water permeable fibers 17a.

Next, a resin is poured into the jig, and the whole of the vicinities of the end portions of the bundle of water permeable fibers 17a is cured by the resin. This operation is performed on both end portions of the bundle of water permeable fibers 17a. By detaching the jig, the bundle of water permeable fibers 17a, the both end portions of which are cured by the resin and which has a cylindrical shape as a whole, is formed. To secure the openings of the water permeable fibers 17a closed by the resin, the bundle of water permeable fibers 17a having the cylindrical shape is cut at a left end portion in parallel with a bottom surface of itself. Thus, the bundle of water permeable fibers 17a (humidifying member 17), having the cylindrical shape as a whole, is formed. In this case, as illustrated in FIG. 2A, a left end portion 17b of the humidifying member 17 is cured by the resin in a state that the one-end openings of the water permeable fibers 17a are opened. It is conceivable that a right end portion 17c of the humidifying member 17 is cured by a resin in a state that the one-end openings of the water permeable fibers 17a are closed. Note that a method for forming the cylindrical humidifying member 17 is not limited to the above-described one but may be another forming method.

As described above, the holder unit 18 is constituted by the holder member 18a and the holder member 18b. As shown in FIGS. 2A and 2B, the holder member 18a is constituted by a circular ring member 18c having a circular ring shape. The inside of the circular ring member 18c is hollow. The circular ring member 18c has a ring opening 18d having the shape of a circular ring, the diameter of which is smaller than the diameter of the circular ring member 18c, in one end surface. Into the ring opening 18d, the end portion of the humidifying member 17 is inserted. A hole 18e is provided in the other end surface of the circular ring member 18c, to receive the liquid supplied from the liquid supply unit 13.

By inserting the humidifying member 17 into the ring opening 18d, the circular ring member 18c holds the humidifying member 17. The liquid supplied from the liquid supply unit 13 is accumulated in a hollow interior 18f of the circular ring member 18c. The left end portion of the humidifying member 17 is present in the hollow interior 18f of the circular ring member 18c. Thus, the liquid penetrates into the bundle of water permeable fibers 17a. To prevent the liquid from leaking from a gap between the ring opening 18d and the humidifying member 17, the gap between the ring opening 18d and the humidifying member 17 is sealed.

As illustrated in FIG. 2A, the holder member 18b is constituted by a circular ring member 18g. The circular ring member 18g has the same structure as the circular ring member 18c, except that the hole 18e is not provided thereto. By inserting the right end portion of the humidifying member 17 into a ring opening 18h formed in the circular ring member 18g, the circular ring member 18g holds the humidifying member 17.

### <1-3. Modification example of holder unit>

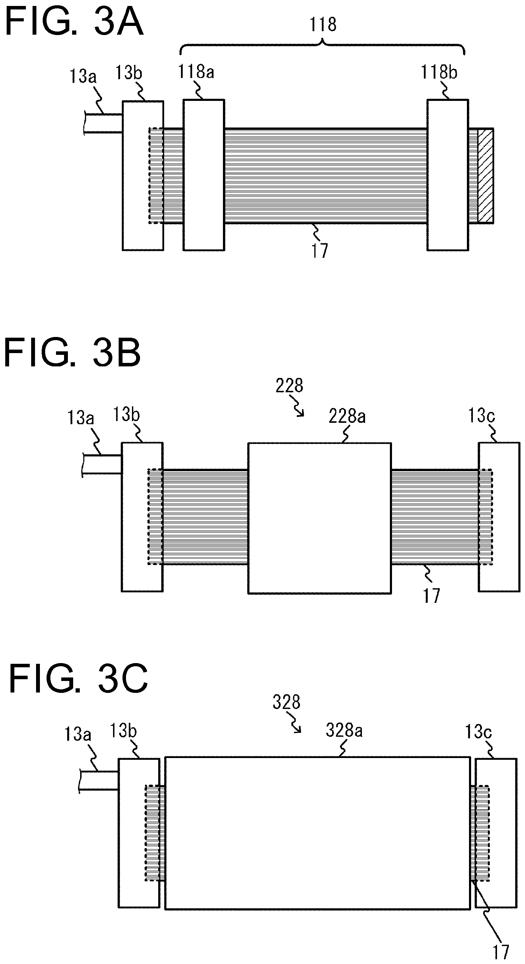

FIG. 3 is a drawing illustrating examples of the structures of holder units 118 to 328 according to other embodiments of the present invention. The holder unit 18 has an aspect that holds the humidifying member 17 at its both ends. The holder unit 18 leads the liquid supplied from the liquid supply unit 13 to the openings of the plurality of water permeable fibers 17a (humidifying member 17). The following holder units according to other embodiments of the present invention have only the function of holding the humidifying member 17. In these cases, a member corresponding to the holder member 18a illustrated in FIGS. 1 and 2 may be regarded as the leading unit 13b that constitutes a part of the liquid supply unit 13, as described above. A member 13c corresponding to the holder member 18b may be regarded as a part of the humidifying member 17, from the viewpoint of closing the other end openings of the water permeable fibers 17a.

FIG. 3A is a drawing illustrating an aspect in which the holder unit 118 holds the humidifying member 17. The holder unit 118 has an aspect that holds the humidifying member 17 at positions that are shifted from the both ends of the humidifying member 17 by a certain distance to the center. The holder unit 118 is constituted by, for example, circular ring members 118a and 118b. The humidifying member 17 is attached to inner peripheries of the circular ring members 118a and 118b. It is conceivable that aspects of the attachment may include an aspect of attaching by gluing, an aspect of attaching using a specific member, an aspect of sandwiching the humidifying member 17 from the inside and outside thereof using another circular ring member, the outer diameter of which is smaller than the inner diameter of the circular ring members 118a and 118b, or the like, and the present invention includes all of the other attachment aspects. The circular ring members 118a and 118b thereby hold the humidifying member 17. Note that the openings at the right end portion of the humidifying member 17 are closed by a process using a resin illustrated in FIG. 2.

FIG. 3B is a drawing illustrating an aspect in which the holder unit 228 holds the humidifying member 17. The holder unit 228 has an aspect that holds the humidifying member 17 in the vicinity of its center. The holder unit 228 is constituted by, for example, a circular ring member 228a. The humidifying member 17 is attached to an inner periphery of the circular ring member 228a. An attachment aspect is the same as that in the case of the circular ring members 118a and 118b. The circular ring member 228a thereby holds the humidifying member 17. Note that the openings at the right end portion of the humidifying member 17 are closed by attaching the member 13c corresponding to the holder member 18b, but may be closed by a process using a resin.

FIG. 3C is a drawing illustrating an aspect in which the holder unit 328 holds the humidifying member 17. The holder unit 328 has an aspect that holds the whole of the humidifying member 17. The holder unit 328 is constituted by, for example, a circular ring member 328a. The circular ring member 328a has an aspect in which the circular ring member 228a is extended in its length. As to the matters other than this, the aforementioned description using FIG. 3B is applicable to the circular ring member 328a.

As described above, the holder unit may have various aspects in the number and lengths of the circular ring members, as long as the holder unit can hold the humidifying member 17. The above-described shape of the circular ring member is just an example, and the circular ring member may have another shape, as long as the circular ring member can hold the humidifying member 17.

### <1-4. Modification examples of humidifying member and holder unit>

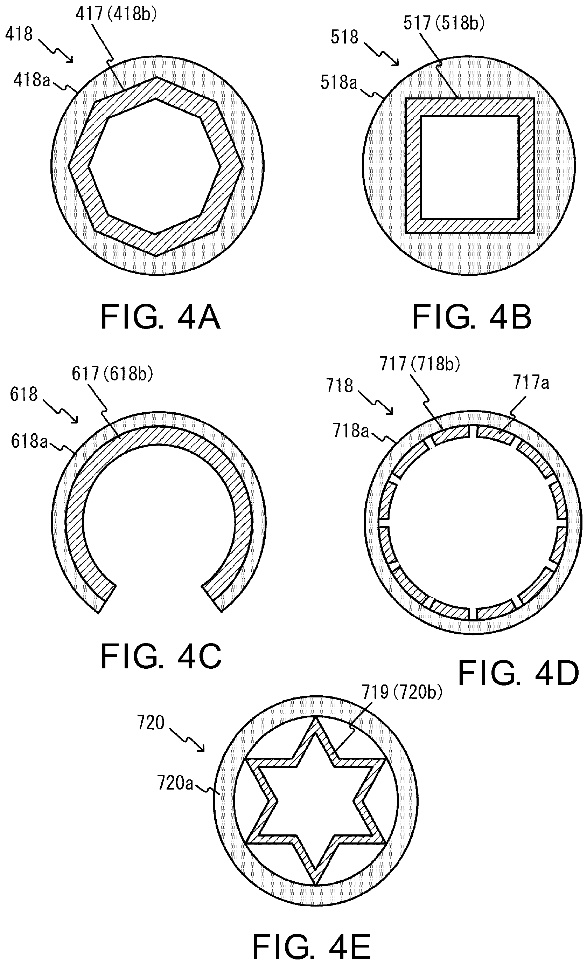

FIG. 4 is a sectional view illustrating examples of structures of humidifying members 417 to 719 and holder units 418 to 720 according to other embodiments of the present invention. In the present invention, the holder unit holds the humidifying member such that the humidifying member is disposed in the shape of a pipe along an inner periphery of the inspiratory pipe in a continuous or discontinuous manner in a circumferential direction. FIGS. 4A and 4B illustrate the humidifying members 417 and 517, each of which has the pipe-shaped aspect along the inner periphery of the inspiratory pipe in a continuous manner in the circumferential direction, and the holder units 418 and 518, respectively.

As illustrated in FIG. 4A, the humidifying member 417 is a water permeable pipe-shaped member that is octagonal in cross section. In the case of the humidifying member 417, a lot of water permeable fibers 17a are inserted into a hollow ring-shaped jig having an octagonal ring-shaped opening in a bottom surface. After that, in the same manner as that described with reference to FIG. 2, a resin is poured to cure the whole of the vicinities of both end portions of a bundle of water permeable fibers 17a by the resin. By detaching the jig, the bundle of water permeable fibers 17a (humidifying member 417) that has an octagonal pipe-shaped shape as a whole is formed.

The holder unit 418 is constituted by a circular ring member 418a that has, for example, an octagonal ring-shaped opening 418b, instead of the ring opening of the circular ring member illustrated in FIGS. 2 and 3. By inserting the humidifying member 417 into the ring opening 418b, the circular ring member 418a holds the humidifying member 417.

As illustrated in FIG. 4B, the humidifying member 517 is a water permeable pipe-shaped member that is rectangular in cross section. The holder unit 518 is constituted by a circular ring member 518a that has, for example, a rectangular ring-shaped opening 518b, instead of the ring opening of the circular ring member illustrated in FIGS. 2 and 3. The description about the humidifying member 417 and the holder unit 418 is applicable to the humidifying member 517 and the holder unit 518, as is, except for having the rectangular shape in cross section, so that a description thereof is omitted here.

As described above, the humidifying member having the pipe-shaped aspect along the inner periphery of the inspiratory pipe in a continuous manner in the circumferential direction has a variety of shapes, but not limited to above, and any pipe-shaped aspect can be applicable to the humidifying member of the present invention. A variety of aspects are applicable to the holder unit of the present invention, in accordance with the aspect of the humidifying member.

FIGS. 4C and 4D illustrate humidifying members 617 and 717, each of which has the pipe-shaped aspect along the inner periphery of the inspiratory pipe 11 in a discontinuous manner in the circumferential direction, and holder units 618 and 718, respectively.

As illustrated in FIG. 4C, the humidifying member 617 is a water permeable pipe-shaped member that is unclosed (partly opened in cross section) circular in cross section. The humidifying member according to the present invention includes the humidifying member of this shape. The holder unit 618 has an aspect such that, for example, a ring opening 618b having a shape corresponding to the shape of the humidifying member 617 is formed in an unclosed (partly opened) circular ring member 618a. By inserting the humidifying member 617 into the ring opening 618b, the circular ring member 618a holds the humidifying member 617. As described above, the unclosed (partly opened) circular shape can be applied to the holder unit.

As illustrated in FIG. 4D, the humidifying member 717 has a circular shape that is discontinuous in cross section as a whole. The humidifying member 717 is formed from a plurality of plane members 717a that are arranged so as to be discontinuously circular in cross section.

The holder unit 718 is constituted by a circular ring member 718a that has, for example, ring openings 718b having a shape corresponding to the discontinuous circular cross section, as described above, instead of the ring-shaped opening illustrated in FIG. 2. By inserting the humidifying member 717 including the plurality of plane members 717a into the ring openings 718b, the circular ring member 718a holds the humidifying member 717.

FIGS. 4C and 4D illustrate the humidifying members that are circular in cross section, out of the humidifying members having the pipe-shaped aspects along the inner peripheries of the inspiratory pipes in a discontinuous manner in the circumferential direction, but not limited to above, and the humidifying member may be polygonal in cross section. In other words, a variety of aspects are applicable to the pipe-shaped humidifying member that extends along the inner periphery of the inspiratory pipe in a discontinuous manner in the circumferential direction. All shapes in which the aforementioned matters are reflected can be applied to the humidifying member and the holder unit according to the present invention.

FIG. 4E illustrates a humidifying member 719 having a pipe-shaped aspect (the shape being a star in cross section) along the inner periphery of the inspiratory pipe 11 in a continuous manner in the circumferential direction, and a holder unit 720. The humidifying member 719 is a pipe-shaped member having the shape of a star in cross section. The humidifying member according to the present invention includes this shape. The holder unit 720 has an aspect such that, for example, a star-shaped opening 720b corresponding to the shape of the humidifying member 719 is formed in a circular ring member 720a. By inserting the humidifying member 719 into the star-shape opening 720b, the circular ring member 720a holds the humidifying member 719. Note that the star-shaped cross section is just an example, and the present invention includes a cross section of another shape.

### <1-5. Modification examples of arrangement of water permeable fibers>

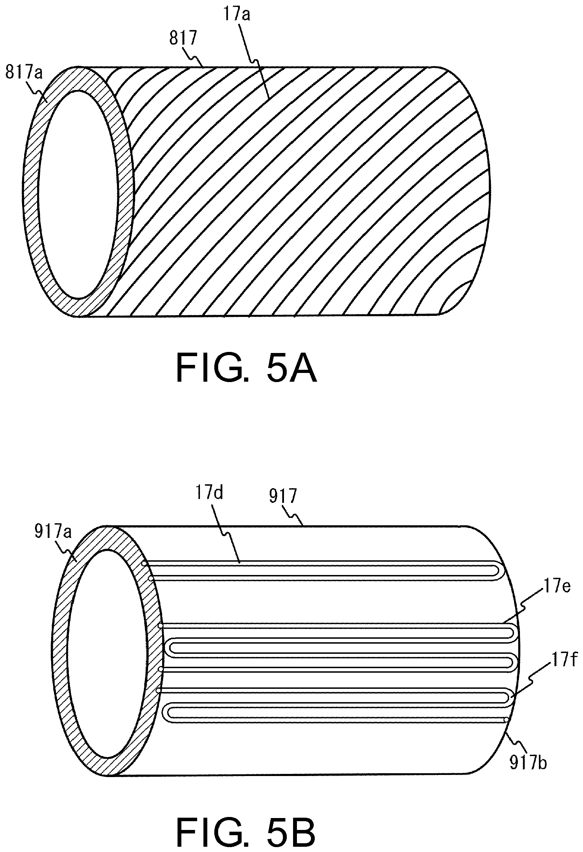

FIG. 5 is a drawing illustrating aspects of humidifying members 817 and 917 according to other embodiments of the present invention. FIG. 5A is a drawing illustrating an aspect of the humidifying member 817. As illustrated in FIG. 1, the humidifying member 17 is formed from many water permeable fibers 17a that are laid straight along the length direction of the inspiratory pipe 11 and formed into the cylindrical shape as a whole. In the humidifying member 817, the water permeable fibers 17a are not laid straight along the length direction of the inspiratory pipe 11. In the humidifying member 817, the water permeable fibers 17a are arranged helically along a curved surface of a cylindrical shape, and formed into a cylindrical shape as a whole. At least inlet openings of the water permeable fibers 17a are disposed in an end surface. On the other hand, outlets of the water permeable fibers 17a, which are disposed on the opposite end surface, are closed. The helical arrangement of the water permeable fibers 17a along the curved surface of the cylindrical shape is just an example, and the water permeable fibers 17a may be arranged in a different aspect along the curved surface of the cylindrical shape.

FIG. 5B is a drawing illustrating an aspect of the humidifying member 917. FIG. 5B illustrates only water permeable fibers 17d to 17f out of the water permeable fibers. In the cylindrical shape constituting the humidifying member 917, the water permeable fiber 17d itself is bent into the shape of the letter U, and disposed such that an inlet and an outlet of the water permeable fiber 17d are disposed on an end surface 917a. In the cylindrical shape constituting the humidifying member 917, the water permeable fiber 17e itself is bent into the shape of the letter W, and disposed such that an inlet and an outlet of the water permeable fiber 17e are disposed on the end surface 917a. In the cylindrical shape constituting the humidifying member 917, the water permeable fiber 17f itself is bent into the shape of the letter N, and disposed such that an inlet is disposed on one end surface 917a, while an outlet is disposed on the other end surface 917b. The present invention includes such an aspect. The above-described aspects are just examples. The present invention includes the water permeable fibers that are bent to various aspects. The present invention also includes the water permeable fibers ,each of inlets and outlets of which is disposed on an end surface, a side surface, or another position.

### <2. Respiratory assistance device 2>

### <2-1. Whole structure of respiratory assistance device 2>

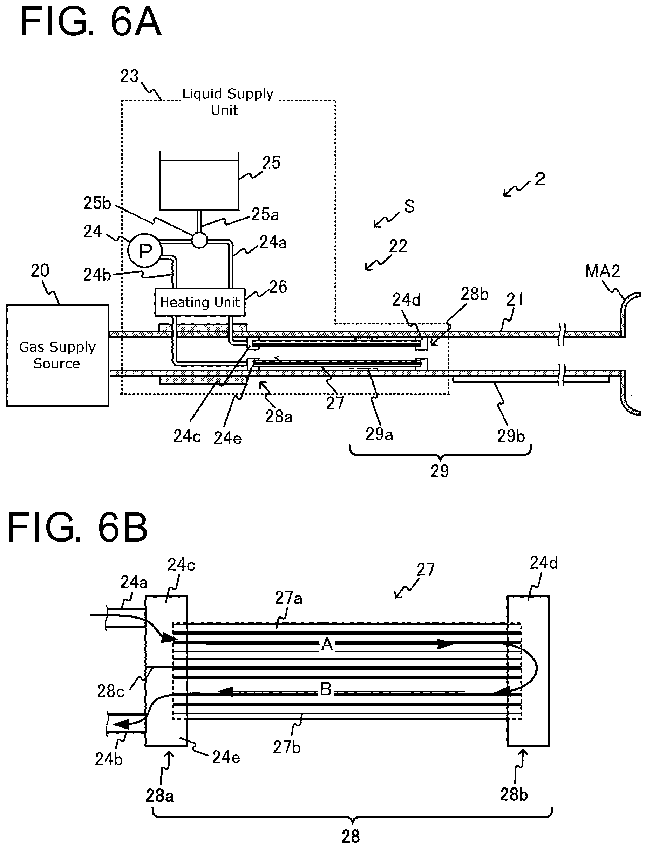

FIG. 6 is a drawing illustrating a respiratory assistance device 2 according to another embodiment of the present invention. FIG. 6A is a sectional view illustrating the whole structure of the respiratory assistance device 2. FIG. 6B is a drawing illustrating a humidifying member 27 and a holder unit 28 in the respiratory assistance device 2.

As with the respiratory assistance device 1, the respiratory assistance device 2 is used by a patient (user) having respiratory disturbance, to feed a gas (compressed air, oxygen, or the like) into an airway of the patient as an intake air. More specifically, the respiratory assistance device 2 includes a gas supply source 20, an inspiratory pipe 21, a humidifier 22, and the like.

Comparing the respiratory assistance device 1 with the respiratory assistance device 2, the gas supply sources and the inspiratory pipes are identical, and the description thereof has already been performed in the description about the respiratory assistance device 1 and hence is omitted. The difference between the respiratory assistance device 1 and the respiratory assistance device 2 includes the structure of the liquid supply unit and an aspect of each portion of the humidifier, owing to the difference in the structure of the liquid supply unit.

The humidifier 22 of the respiratory assistance device 2 humidifies the gas inside the inspiratory pipe 21, in the same manner as the humidifier 12. To be more specific, the humidifier 22 includes, for example, a liquid supply unit 23, a humidifying member 27, a holder unit 28, a heating unit 29, and the like.

The liquid supply unit 23 for supplying liquid for the humidifying member 27, includes, for example, a pump 24, a supply tube 24a, a return tube 24b, a leading unit including leading members 24c to 24e, a liquid replenishing unit 25, a heating unit 26, and the like. Note that in the following description, it is assumed that the humidifying member 27 is formed from a plurality of water permeable fibers into an aspect as illustrated in FIG. 2.

The pump 24 circulates the liquid in a liquid circulation system S that is constituted by the supply tube 24a and the return tube 24b, and the leading unit including the leading members 24c to 24e, and the humidifying member 27. By the circulation, the pump 24 supplies the liquid to the plurality of water permeable fibers constituting the humidifying member 27. For example, a roller pump is conceivable as the pump 24, but not limited to this, and another pump may be used. Each portion of the liquid supply unit 23 will be described below.

The supply tube 24a supplies the liquid to the humidifying member 27 through the leading member 24c. Through the humidifying member 27 and the leading members 24c to 24e, the liquid flows in the directions of arrows A and B of FIG. 6B. In other words, the leading members 24c to 24e form a U-shaped tube, together with the humidifying member 27. The leading member 24c corresponding to an inlet of the U-shaped tube is connected to the supply tube 24a. The leading member 24d connects an upper half member 27a, i.e., an upper half of the humidifying member 27, to a lower half member 27b, i.e., a lower half of the humidifying member 27. The leading member 24e corresponding to an outlet of the U-shaped tube is connected to the return tube 24b. Accordingly, the liquid supplied from the supply tube 24a flows through the leading member 24c, the upper half member 27a, the leading member 24d, the lower half member 27b, and the leading member 24e, in this order, to the return tube 24b.

Note that, the water permeable fibers constituting the humidifying member 27 and the leading unit, i.e., a group of the leading members, form the U-shaped tube in the aforementioned description, but not limited to this, and may form another aspect. In other words, the water permeable fibers and the leading unit may form any aspect, as long as the aspect is a reciprocating tube. For example, water permeable fibers, such as the water permeable fibers 17d or the water permeable fibers 17e, as illustrated in FIG. 5B, may be bent into a reciprocating aspect to form a reciprocating tube. In this case, a member corresponding to the leading member 24d is omitted.

When the liquid is reduced in the liquid circulation system S, the liquid replenishing unit 25 replenishes the liquid circulation system S with the liquid held by itself. The liquid flowing through the humidifying member 27 is vaporized and diffused into the inspiratory pipe 21. In this process, the liquid flowing through the liquid circulation system S is reduced. The liquid replenishing unit 25 replenishes with the liquid through a replenishing tube 25a. The replenishing tube 25a is connected to the supply tube 24a through, for example, a three-way valve 25b.

The three-way valve 25b opens and closes by, for example, the following aspect. The three-way valve 25b prevents a flow from the supply tube 24a to the replenishing tube 25a. For example, when liquid flowing through the liquid circulation system S is reduced and a pressure inside the supply tube 24a becomes lower than a predetermined threshold value, the three-way valve 25b allows a flow from the replenishing tube 25a to the supply tube 24a. Note that, in FIG. 6A, the replenishing tube 25a is connected to the supply tube 24a, but this is just an example, and the replenishing tube 25a may be connected to the return tube 24b. The opening and closing aspect of the three-way valve 25b may be another aspect, as long as the liquid can be replenished with in accordance with a reduction in the liquid flowing through the liquid circulation system S.

The heating unit 26 heats the liquid in the liquid circulation system S. The liquid heated by the heating unit 26 heats the inside of the inspiratory pipe 21.

The humidifying member 27 vaporizes the liquid supplied from the liquid supply unit 23. The humidifying member 27 is identical to the humidifying member illustrated in FIGS. 1 to 4, and the aforementioned description can be applied to the humidifying member 27, so that a description thereof is omitted.

The holder unit 28 holds the humidifying member 27 such that the humidifying member 27 is disposed in the shape of a pipe along the inner periphery of the inspiratory pipe 21 of the respiratory assistance device 2 in a continuous or discontinuous manner in the circumferential direction. As illustrated in FIG. 6B, the holder unit 28 is constituted by a holder member 28a and a holder member 28b.

The inside of the holder member 28a is hollow. The holder member 28a is partitioned by a partition plate 28c in the vicinity of the center of the inside into two chambers, i.e., upper and lower chambers. In this embodiment, the upper chamber of the holder member 28a is the above-described leading member 24c. The leading member 24c is connected to the supply tube 24a. As illustrated in FIG. 6B, the upper half member 27a of the humidifying member 27 is inserted into the leading member 24c. In the upper half member 27a, openings of the water permeable fibers are opened at both end surfaces. The liquid supplied from the supply tube 24a is accumulated in the leading member 24c. The liquid flows through the upper half member 27a to the direction of the arrow A. In other words, the leading member 24c leads the liquid supplied from the supply tube 24a to the end portion openings of the upper half member 27a.

On the other hand, the inside of the holder member 28b is hollow. In this embodiment, the holder member 28b also serves as the leading member 24d. As illustrated in FIG. 6B, the whole of a right end portion of the humidifying member 27 is inserted into the leading member 24d. In a right end surface of the humidifying member 27, openings of the water permeable fibers are opened. In other words, the leading member 24d serves as a connection tube for connecting a right end portion of the upper half member 27a to a right end portion of the lower half member 27b. Thus, the liquid that has flowed through the upper half member 27a is ejected and accumulated in the leading member 24d. The liquid accumulated in the leading member 24d flows through the lower half member 27b of the humidifying member 27 to the direction of the arrow B. In other words, the leading member 24d leads the liquid ejected from the end portion openings of the upper half member 27a to end portion openings of the lower half member 27b.

The lower chamber of the holder member 28a is the leading member 24e. The liquid that has flowed through the lower half member 27b is ejected and accumulated in the leading member 24e. The leading member 24e is connected to the return tube 24b. The liquid ejected in the leading member 24e flows into the return tube 24b. In other words, the leading member 24e leads the liquid ejected from the end portion openings of the lower half member 27b into the return tube 24b. The liquid flowing through the return tube 24b is further fed into the supply tube 24a by the pump 24. The liquid circulates in this manner.

In the above-described respiratory assistance device 2 according to the embodiment of the present invention, the holder members and the leading members integrally function, but the present invention is not limited to this. In other words, in this embodiment, the scope of the present invention includes an aspect in which the holder members and the leading members are clearly differentiated into two types of components, as illustrated in FIG. 3.

### <2-2. Humidifying member 27 and holder unit 28>

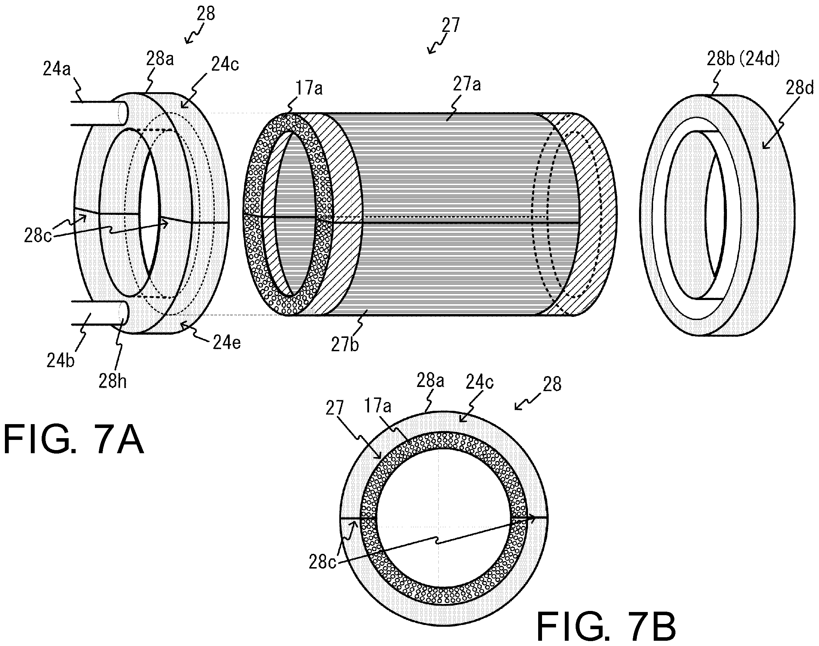

FIG. 7 is a drawing illustrating the humidifying member 27 and the holder unit 28 according to the other embodiment of the present invention. FIG. 7A is a perspective view of the humidifying member 27 and the holder unit 28. FIG. 7B is a sectional view in which the humidifying member 27 is attached to the holder unit 28.

As illustrated in FIG. 7A, the humidifying member 27 is constituted by a lot of water permeable fibers 17a that are bundled in a length direction into a cylindrical shape. An example of the method for forming the humidifying member 27 has already been described in the description about the humidifying member 17 using FIG. 2, and the description of the humidifying member 17 can be applied to the humidifying member 27, so that a description about the humidifying member 27 is omitted.

As illustrated in FIGS. 7A and 7B, the holder members 28a and 28b constituting the holder unit 28 have almost the same structure as the holder members 18a and 18b constituting the holder unit 18 (see FIG. 2). In other words, the holder members 28a and 28b are circular ring-shaped members. The difference between the holder member 18a and the holder member 28a is that, while the inside of the holder member 28a is divided into the leading member 24c and the leading member 24e, the inside of the holder member 18a is not divided in this manner. In other words, to make the holder member 18a constituting the holder unit 18 into the holder member 28a constituting the holder unit 28, partition plates 28c that completely partition the inside of the holder member 18a into upper and lower portions are provided, and a hole 28h is provided for connection to the return tube 24b.

The inside of the holder member 28a is divided into the leading member 24c and the leading member 24e, for the purpose of dividing between a flow of the liquid from the supply tube 24a and a flow of the liquid from the return tube 24b before the liquid enters the inside of the humidifying member 27. By this division, the holder member 28a, the humidifying member 27, and the holder member 28b can constitute a reciprocating tube (for example, a U-shaped tube). Note that, to realize the above-described structure, both of inlet and outlet openings of the water permeable fibers 17a at both end portions of the humidifying member 27 need to be opened.

The holder member 28b constituting the holder unit 28 leads the liquid ejected from an outlet of the upper half member 27a into an inlet of the lower half member 27b, as the leading member 24d. Thus, a certain space 28d is necessary inside the holder member 28b, as a liquid lead path.

As illustrated in FIG. 3, the holder unit 28 may not have the function of holding the humidifying member 27 and the function of leading the liquid at the same time. In other words, separate members may have the function of holding the humidifying member 27 and the function of leading the liquid, respectively. In this case, the members corresponding to the holder member 28a and the holder member 28b illustrated in FIGS. 6 and 7 may be regarded as components of the liquid supply unit 23 having the function of leading the liquid. Another additional member having, for example, the same aspect as illustrated in FIG. 3 may serve as a member having the function of holding the humidifying member 27.

Note that the inlets and outlets of the water permeable fibers constituting the humidifying member may be disposed in any manner, as long as the humidifying member can be disposed in the shape of a pipe along the inner periphery of the inspiratory pipe in a continuous or discontinuous manner in the circumferential direction. The leading unit constituting the liquid supply unit is conceivable to have various aspects, depending on the disposition of the inlets and outlets of the water permeable fibers constituting the humidifying member, and the present invention includes such aspects.

### <3. Respiratory assistance device 3>

FIG. 8 is a drawing illustrating a respiratory assistance device 3 according to another embodiment of the present invention. FIG. 8A is a sectional view illustrating the whole structure of the respiratory assistance device 3. FIG. 8B is a drawing illustrating a humidifying member 37 soaked in a tank 900.

As with the respiratory assistance device 1, the respiratory assistance device 3 is used by a patient (user) having respiratory disturbance, to feed a gas (compressed air, oxygen, or the like) into an airway of the patient as an intake air. More specifically, the respiratory assistance device 3 includes a gas supply source 30, an inspiratory pipe 31, a humidifier 32, and the like.

Comparing the respiratory assistance device 1 with the respiratory assistance device 3, the gas supply sources and the inspiratory pipes are identical, and the description thereof has already been performed in the description about the respiratory assistance device 1 and hence is omitted. The difference between the respiratory assistance device 1 and the respiratory assistance device 3 includes the presence or absence of a liquid supply unit, and the material of a humidifying member.

The humidifier 32 of the respiratory assistance device 3 humidifies the gas inside the inspiratory pipe 31 in the same manner as the humidifier 12. To be more specific, the humidifier 32 includes, for example, a humidifying member 37, a holder unit 38, a heating unit 39, and the like. The humidifying member 37, the holder unit 38, and the heating unit 39 have the same aspects as those of the humidifying member 17, the holder unit 18, and the heating unit 19. Note that, for the sake of simplicity of the drawing, the aspect of the heating unit 39 omits the heating units 19b and 19c of the heating unit 19, but this is just an example.

The respiratory assistance device 1 and the respiratory assistance device 3 have different liquid supply aspects for the humidifying members (humidifying member 17 and humidifying member 37). The liquid supply aspect for the humidifying member 37 will be described below. The humidifying member 37 is formed from a water-absorbing material. Examples of the water-absorbing material may include woven cloth, nonwoven cloth, a synthetic resin such as sponge, and a water-absorbing polymer. The humidifying member 37 may be formed from one type of water-absorbing material, or a plurality of types of water-absorbing materials. In other words, the humidifying member 37 may be formed from, for example, any one selected from among woven cloth, nonwoven cloth, a synthetic resin such as sponge, a water-absorbing polymer, and the like. The humidifying member 37 may be formed from, for example, any two selected from among woven cloth, nonwoven cloth, a synthetic resin such as sponge, a water-absorbing polymer, and the like. The humidifying member 37 may be formed from, for example, all of woven cloth, nonwoven cloth, a synthetic resin such as sponge, a water-absorbing polymer, and the like. What these materials are disposed in the shape of a pipe along the inner periphery of the inspiratory pipe 31 in a continuous or discontinuous manner in the circumferential direction,is the humidifying member 37.

To supply a liquid to the humidifying member 37, for example, as illustrated in FIG. 8B, the humidifying member 37 is soaked into the tank 900 containing water. Thus, the humidifying member 37 absorbs the water and becomes a state of holding moisture. As illustrated in FIG. 8A, the humidifying member 37 is attached to the inside of the inspiratory pipe 31. Whenever the water of the humidifying member 37 is evaporated and the humidifying member 37 becomes a dried state, the humidifying member 37 is replenished with water by the tank 900. Note that the water replenishing aspect for the humidifying member 37 may be another one.

Note that, when the humidifying member 37 is formed from the water-absorbing material, as described about the respiratory assistance device 3, the matters of the respiratory assistance devices 1 and 2 according to the embodiments of the present invention can be appropriately applied, and the present invention includes such applications.

### <4. Respiratory assistance device 4>

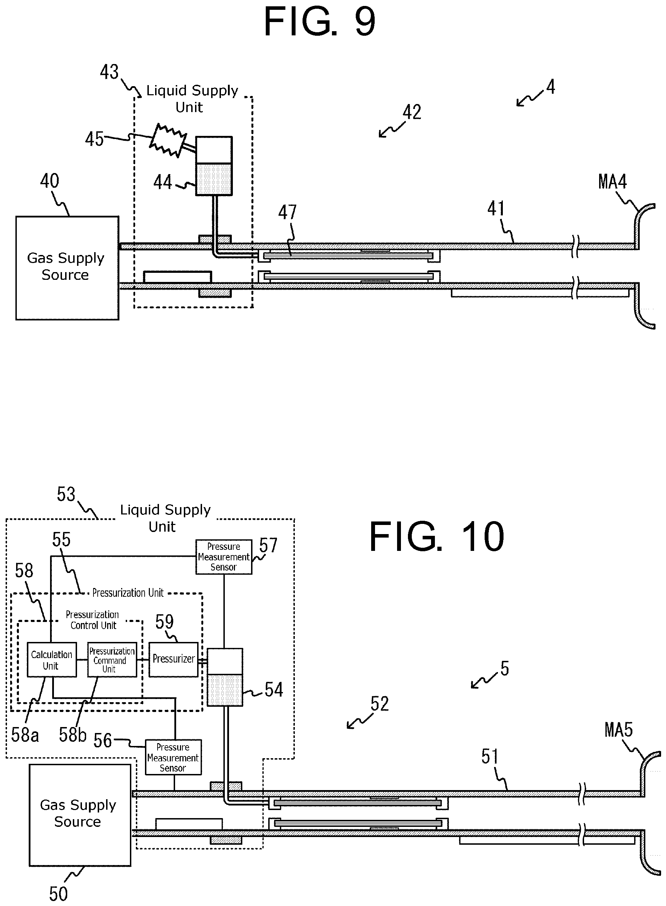

FIG. 9 is a drawing illustrating a respiratory assistance device 4 according to another embodiment of the present invention. The respiratory assistance device 4 is used by a patient (user) having respiratory disturbance, to feed a gas (compressed air, oxygen, or the like) into an airway of the patient as an intake air. More specifically, as illustrated in FIG. 9, the respiratory assistance device 4 includes a gas supply source 40, an inspiratory pipe 41, a humidifier 42, and the like.

Comparing the respiratory assistance device 1 with the respiratory assistance device 4, the gas supply sources and the inspiratory pipes are identical, and the description thereof has already been performed in the description about the respiratory assistance device 1 and hence is omitted. The difference between the respiratory assistance device 1 and the respiratory assistance device 4 includes the structure of a liquid supply unit constituting a humidifier.

A liquid supply unit 43 of the respiratory assistance device 4 includes a container 44 and a pressurization unit 45. The container 44 holds a liquid therein. The pressurization unit 45 applies a pressure to the inside of the container 44. By way of example, as illustrated in FIG. 9, the container 44 is formed from, for example, an accordion member having stretchability (expandability and retractability). For example, when the accordion member is manually retracted, air inside the accordion member is pushed out into the container 44. Thus, a pressure is applied to the inside of the container 44. Repeating expansion and retraction of the accordion member further applies a pressure to the inside of the container 44.

Note that, the aspect of the pressurization unit 45 is not limited to the accordion member, as long as the pressurization unit 45 is formed from a material that can apply a pressure, and may be formed from, for example, a hollow rubber member. In the case of the hollow rubber member, for example, the hollow rubber member is gripped and stretched with a hand, so as to push out air inside the hollow rubber member into the container 44. Thus, a pressure is applied to the inside of the container 44.

Next, a role of the pressurization unit 45 will be described. When a gas supply source 40 supplies a gas into an inspiratory pipe 41, the pressure of the inside of the inspiratory pipe 41 is increased. Accordingly, the pressure of the inside of the container 44 is relatively reduced, as compared with the pressure of the inside of the inspiratory pipe 41. When the pressure of the inside of the container 44 is lower than the pressure of the inside of the inspiratory pipe 41, the liquid supplied from the container 44 to a humidifying member 47 flows back, owing to the difference in pressure. To prevent this situation, the pressurization unit 45 applies a pressure to the inside of the container 44. Increasing the pressure of the inside of the container 44 to a higher than the pressure of the inside of the inspiratory pipe 41 can prevent the aforementioned backflow.

In the aforementioned description, the member is manually stretched to apply a pressure to the inside of the container 44, but the present invention is not limited to this. An example thereof will be described below as a respiratory assistance device 5, with reference to FIG. 10.

### <5. Respiratory assistance device 5>

FIG. 10 is a drawing illustrating a respiratory assistance device 5 according to another embodiment of the present invention. In the respiratory assistance device 5, the manual pressurization aspect to apply a pressure to the inside of the container 44 in the respiratory assistance device 4 is modified into an automatic pressurization aspect. The other portions of the respiratory assistance device 5 are the same as those of the respiratory assistance device 4, and the description about those of the respiratory assistance device 4 can be applied as is, so that the description for the other portions of the respiratory assistance device 5 is omitted.

The pressurization unit 55 of the respiratory assistance device 5 includes, for example, pressure measurement sensors 56 and 57, a pressurization control unit 58, and a pressurizer 59. The pressure measurement sensor 56 measures a pressure P1 of the inside of an inspiratory pipe 51. The pressure measurement sensor 57 measures a pressure P2 of the inside of a container 54. The pressurization control unit 58 controls a pressurization operation of the pressurizer 59, on the basis of a measurement result of the pressure measurement sensor 56 and/or the pressure measurement sensor 57. Under the control of the pressurization control unit 58, the pressurizer 59 performs the pressurization operation on the inside of the container 54.

The pressurization control unit 58 is assumed to have an aspect including a calculation unit 58a and a pressurization command unit 58b, as an example. The calculation unit 58a calculates a pressure difference (P2 - P1) from measurement values of the pressure measurement sensors 56 and 57. When the pressure difference (P2 - P1) is equal to or lower than a predetermined threshold value (for example, P2 - P1 < 0), the pressurization command unit 58b commands the pressurizer 59 to apply a pressure.

The pressurization unit 55 of the respiratory assistance device 5 may be constituted by, for example, the pressure measurement sensor 56, the pressurization control unit 58, and the pressurizer 59. In this case, when the pressure P1 of the inside of the inspiratory pipe 51 is higher than a predetermined threshold value, the pressurization control unit 58 commands the pressurizer 59 to apply a pressure. In this case, the predetermined threshold value and how much pressure the pressurizer 59 applies are assumed to be determined so as to prevent the aforementioned flowback, on the basis of, for example, variation data of the pressure P1 of the inside of the inspiratory pipe 51 with time, variation data of the pressure P2 of the inside of the container 54 with time, and the like when the respiratory assistance device 5 is operated.

The pressurization unit 55 of the respiratory assistance device 5 may be constituted by, for example, the pressure measurement sensor 57, the pressurization control unit 58, and the pressurizer 59. In this case, when the pressure P2 of the inside of the container 54 is lower than a predetermined threshold value, the pressurization control unit 58 commands the pressurizer 59 to apply a pressure. In this case, the predetermined threshold value and how much pressure the pressurizer 59 applies are assumed to be determined so as to prevent the aforementioned flowback, on the basis of, for example, variation data of the pressure P1 of the inside of the inspiratory pipe 51 with time, variation data of the pressure P2 of the inside of the container 54 with time, and the like when the respiratory assistance device 5 is operated.

The pressurization unit 55 of the respiratory assistance device 5 may be constituted by, for example, the pressurization control unit 58 and the pressurizer 59. In this case, the pressurization control unit 58 commands the pressurizer 59 to apply a pressure at, for example, predefined time intervals. The time intervals at which the pressurization control unit 58 issues the pressurization commands are determined on the basis of, for example, variation data of the pressure difference with time and the like when the respiratory assistance device 5 is operated.

According to the respiratory assistance devices 4 and 5 according to the embodiments of the present invention, it is possible to prevent the flowback owing to the difference in pressure between the inside of the inspiratory pipe 41 and the inside of the container 44, and between the inside of the inspiratory pipe 51 and the inside of the container 54.

### <6. Respiratory assistance device 6>

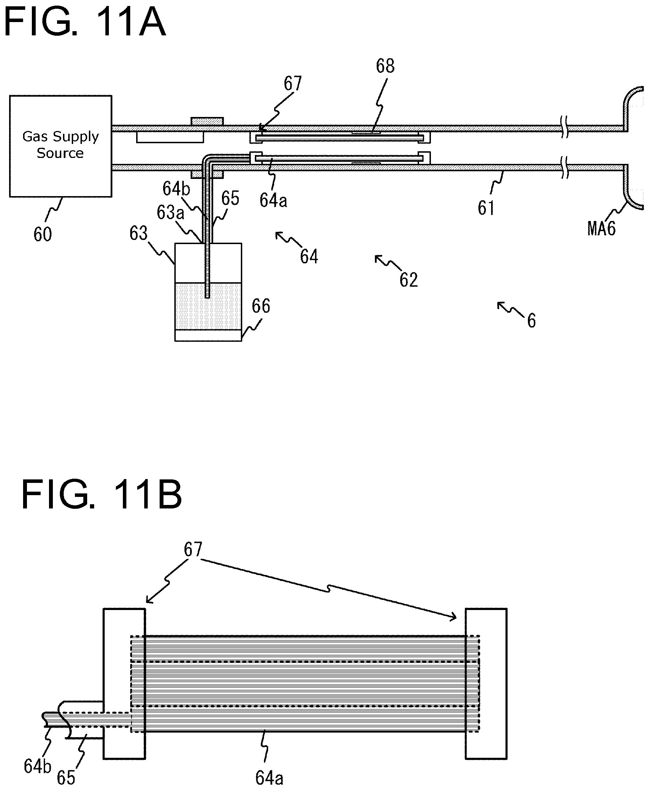

FIG. 11 is a drawing illustrating a respiratory assistance device 6 according to another embodiment of the present invention. FIG. 11A is a sectional view illustrating the whole structure of the respiratory assistance device 6. FIG. 11B is a drawing illustrating the vicinity of a humidifying member 64a and a holder unit 67 in the respiratory assistance device 6. As with the respiratory assistance device 1, the respiratory assistance device 6 is used by a patient (user) having respiratory disturbance, to feed a gas (compressed air, oxygen, or the like) into an airway of the patient as an intake air. More specifically, as illustrated in FIG. 11, the respiratory assistance device 6 includes a gas supply source 60, an inspiratory pipe 61, a humidifier 62, and the like.

Comparing the respiratory assistance device 1 with the respiratory assistance device 6, the gas supply sources and the inspiratory pipes are identical, and the description thereof has already been performed in the description about the respiratory assistance device 1 and hence is omitted. The difference between the respiratory assistance device 1 and the respiratory assistance device 6 includes a liquid supply aspect to supply a liquid into the humidifying member. This will be described below.

The humidifier 62 of the respiratory assistance device 6, just as with the humidifier 12, humidifies a gas inside the inspiratory pipe 61. To be more specific, the humidifier 62 includes, for example, a container 63, a humidifying member 64, a guide member 65, a heating unit 66, a holder unit 67, a heating unit 68, and the like. Note that the holder unit 67 and the heating unit 68 are identical to the holder unit 18 and the heating unit 19 of the respiratory assistance device 1, respectively, and the description about the holder unit 18 and the heating unit 19 is applicable to the holder unit 67 and the heating unit 68, as is, so that a description thereof is omitted here.

The container 63 is a vessel for containing a required amount (for example, 60 cc for one night (8 hours)) of a liquid (water or the like). The container 14 is preferably closed in an airtight manner.

The humidifying member 64 is disposed in the inspiratory pipe 61 in a state that a part (for example, an end portion) of itself is inserted in the container 63. When a liquid is contained in the container 63, the part of the humidifying member 64 is immersed in the liquid in the container 63. To be more specific, the humidifying member 64 is constituted by a humidifying member 64a and a humidifying member 64b. As illustrated in FIG. 11, the humidifying member 64a, out of the humidifying member 64 disposed inside the inspiratory pipe 61, refers to an uncovered portion that is not covered with the guide member 65. As illustrated in FIG. 11, the humidifying member 64b out of the humidifying member 64 refers to a portion from a point inserted into the container 63 (a portion immersed in the contained liquid in FIG. 11) to a terminal end point of the guide member 65.

The humidifying member 64a and the humidifying member 64b are assumed to be formed integrally with each other. However, the present invention is not limited to this, but the humidifying member 64a and the humidifying member 64b may be formed as separate members and connected to each other.

The humidifying member 64b absorbs the liquid inside the container 63 from its end portion, and allows the liquid to penetrate to the humidifying member 64a by, for example, capillarity. The liquid penetrated to the humidifying member 64a is naturally vaporized inside the inspiratory pipe 61.

The humidifying member 64 is assumed to be formed from a water-absorbing material that can absorbs the liquid inside the container 63. Examples of the water-absorbing material may include a material that can absorb liquid by capillarity, and a water-absorbing polymer that can absorb liquid by another factor. Examples of the material that can absorb liquid by capillarity may include a porous material. Examples of the porous material may include woven cloth, nonwoven cloth, a material formed from hollow fibers, a porous synthetic resin such as sponge, and a porous water-absorbing polymer. The plurality of types of porous materials are present, as described above, and the humidifying member 64 may be formed from one type of porous material, or a plurality of types of porous materials. In other words, the humidifying member 64 may be formed from at least one of the aforementioned materials.

The guide member 65 guides the humidifying member 64b from an outlet 63a of the container 63 to the inside of the inspiratory pipe 61. The guide member 65 preferably has an aspect that covers the periphery of the humidifying member 64b in order to prevent the liquid contained in the humidifying member 64b from vaporizing outside the inspiratory pipe 61. A connection portion between the outlet 63a of the container 63 and the guide member 65 is preferably sealed in order to prevent leakage of the inside liquid.

The heating unit 66 heats the liquid inside the container 63. Increasing the temperature of the liquid inside the container 63 facilitates vaporizing the liquid from the humidifying member 64a.

The above-described respiratory assistance device 6 has the simple structure and ease of use. Therefore, it is possible to reduce the manufacturing cost of the respiratory assistance device 6.

### <7. Respiratory assistance device 7>

### <7-1. Whole structure of respiratory assistance device 7>

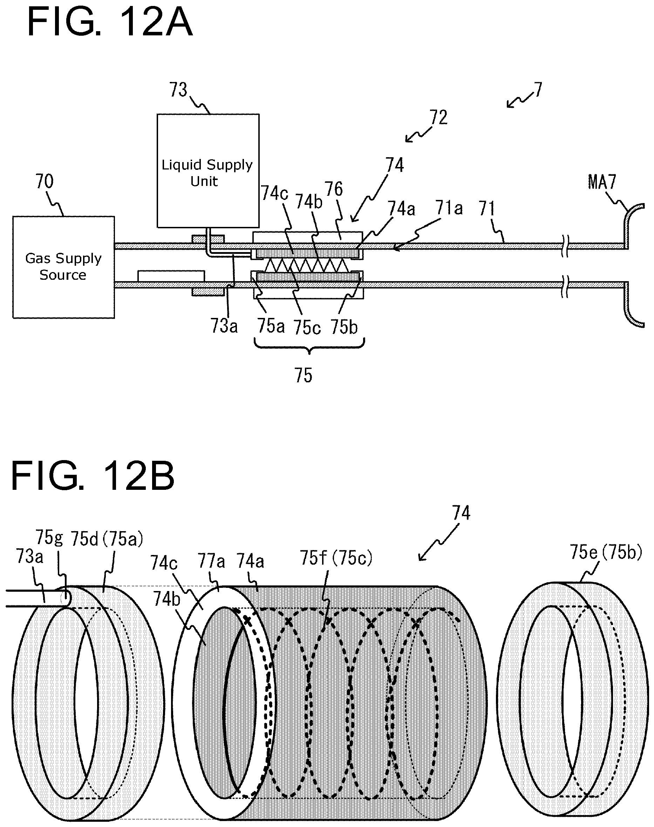

FIG. 12 is a drawing illustrating a respiratory assistance device 7 according to another embodiment of the present invention. FIG. 12A is a sectional view illustrating the whole structure of the respiratory assistance device 7. FIG. 12B is a drawing illustrating the vicinity of a humidifying member 74 and a holder unit 75 in the respiratory assistance device 7.

As illustrated in FIG. 12A, the respiratory assistance device 7 includes a gas supply source 70, an inspiratory pipe 71, a humidifier 72, and the like.

Comparing the respiratory assistance device 1 with the respiratory assistance device 7, the gas supply sources and the inspiratory pipes are identical, and the description thereof has already been performed in the description about the respiratory assistance device 1 and hence is omitted. The difference between the respiratory assistance device 1 and the respiratory assistance device 7 includes an aspect of a humidifying member. This will be described below.

As with the humidifier 12, the humidifier 72 of the respiratory assistance device 7 humidifies a gas inside the inspiratory pipe 71. To be more specific, the humidifier 72 includes, for example, a liquid supply unit 73, a humidifying member 74, a holder unit 75, a heating unit 76, and the like. Note that, as the liquid supply unit 73, any of the above-described liquid supply units may be adopted.

The liquid supply unit 73 supplies a liquid through a supply tube 73a to the humidifying member 74.

The humidifying member 74 vaporizes the liquid supplied from the liquid supply unit 73. As with the humidifying member 17, the humidifying member 74 has the pipe-shaped aspect along an inner periphery of the inspiratory pipe 71 in a continuous or discontinuous manner in a circumferential direction. To be more specific, in the humidifying member 74, as illustrated in FIG. 12B, its own pipe-shape outer peripheral surface 74a is formed of an inner peripheral surface 71a of the inspiratory pipe 71.

In the humidifying member 74, as illustrated in FIG. 12B, the other pipe-shape forming portions, including its own pipe-shape inner peripheral surface 74b and the like, are formed from a material that allows passage of gases, while preventing passage of liquids. As such a material, for example, any of silicone rubber and all types of materials subjected to a moisture permeable waterproof process is conceivable, by way of example, but the present invention is not limited to this. A liquid supplied from the liquid supply unit 73 is held in an interior space 74c formed between the inner peripheral surface 71a of the inspiratory pipe 71 and the other pipe-shape forming portions. Note that the own pipe-shape outer peripheral surface 74a may be formed from a material that allows passage of gases, while preventing passage of liquids. In other words, the whole of the humidifying member 74 may be formed from a material that allows passage of gases, while preventing passage of liquids.

The holder unit 75 holds the humidifying member 74. The holder unit 75 is constituted by, for example, holder members 75a to 75c. The holder members 75a and 75b hold the humidifying member 74 in a state of closing both end portions of the humidifying member 74. As illustrated in FIG. 12B, the holder members 75a and 75b are conceivable to be, for example, circular ring members 75d and 75e having circular ring shapes. The inside of the circular ring members 75d and 75e is hollow. Each of the circular ring members 75d and 75e is opened at one end surface. Both ends of the humidifying member 74 are inserted into the opened end surfaces. Thus, the humidifying member 74 is held in a state of being closed at both ends with the holder members 75d and 75e. Note that, when there are gaps between the circular ring member 75d and the humidifying member 74 and between the circular ring member 75e and the humidifying member 74, the gaps between the circular ring member 75d and the humidifying member 74 and between the circular ring member 75e and the humidifying member 74 are sealed to prevent leaking of the liquid from the gaps. Note that the circular ring members 75d and 75e may be not necessarily used, as long as the end portions of the humidifying member 74 are closed.

A hole 75g is provided in the other end surface of the circular ring member 75d. The hole 75g is connected to the supply tube 73a. The liquid, which is supplied from the liquid supply unit 73 through the supply tube 73a, is supplied through the circular ring member 75d to the interior space 74c of the humidifying member 74.

As shown in FIGS. 12A and 12B, for example, the holder member 75c may be a helical member 75f having a helical shape. As the helical member 75f, a helical spring member is conceivable, by way of example, but the present invention is not limited to this.

When the helical member 75f is disposed such that an axis of the helical member 75f and an axis of the humidifying member 74 coincide with each other, the helical member 75f may have such a diameter that, for example, an outer periphery of the helical member 75f is in contact with an inner periphery of the humidifying member 74. Since the helical member 75f thereby supports the humidifying member 74 from the side of the pipe-shape inner peripheral surface 74b, even if the pipe-shape inner peripheral surface 74b of the humidifying member 74 is formed from a soft sheet material, a load on the pipe-shape inner peripheral surface 74b, owing to the weight of the liquid held inside the humidifying member 74, can be reduced.

The holder member 75c is not limited to the helical member, but may have another shape, as long as the holder member 75c can support the humidifying member 74 from the side of the pipe-shape inner peripheral surface 74b, as described above. For example, the holder member 75c may be a ring continuation member in which circular rings having such diameters as to be in contact with the pipe-shape inner peripheral surface 74b of the humidifying member 74 are arranged at predefined intervals, and the adjoining circular rings are connected to each other. The holder member 75c may be a ring continuation member in which polygonal rings that are in contact with the pipe-shape inner peripheral surface 74b of the humidifying member 74 are arranged at predefined intervals, and the adjoining polygonal rings are connected to each other. The predefined intervals may be any of regular intervals and irregular intervals.

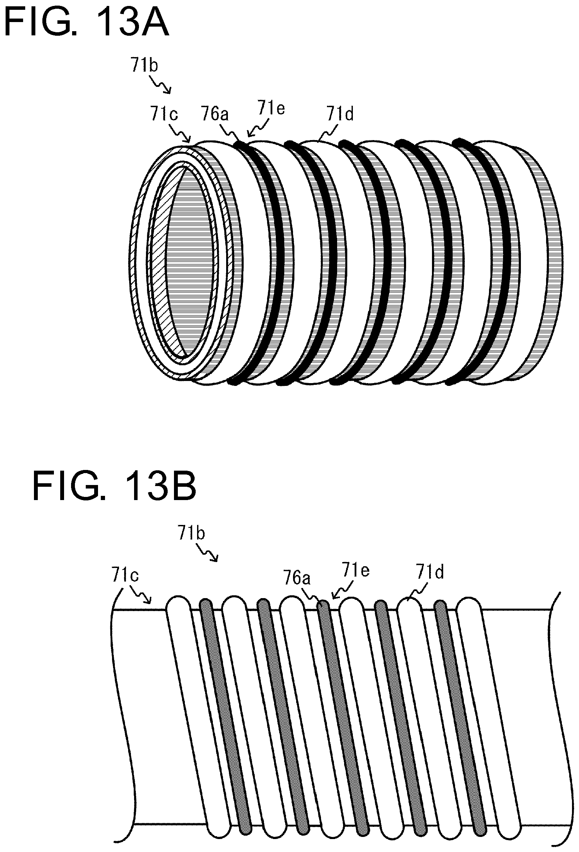

The heating unit 76 is disposed on an outer periphery of the inspiratory pipe 71. The heating unit 76 heats the humidifying member 74 disposed in the expiratory pipe 71 from the side of the outer periphery of the inspiratory pipe 71. Therefore, the liquid contained in the humidifying member 74 is easily vaporized. An aspect of the heating unit 76 will be further described with reference to FIG. 13 later.

As described above, disposing the humidifying member 74 inside the expiratory pipe 71 allows uniformly humidifying the gas inside the inspiratory pipe 71 of the respiratory assistance device 7. The humidifying member 74 is disposed such that intake air passes through the inside of itself, and therefore has a large humidification area for the intake air. Therefore, the humidifying member 74 can sufficiently humidify the intake air.

### <7-2. Modification examples of expiratory pipe 71 and heating unit 76>

FIG. 13 is a drawing illustrating the state of using an accordion pipe 71b as the inspiratory pipe 71. FIG. 13A is a perspective view of the accordion pipe 71b on which a heating unit 76a is disposed. FIG. 13B is a plan view of the accordion pipe 71b on which the heating unit 76a is disposed.

For example, as shown in FIGS. 13A and 13B, the accordion pipe 71b may have an aspect in which a projection portion 71d is formed in an outer peripheral surface of itself in a helical manner. In the case of such an accordion pipe 71b, a depression portion 71e is formed in a helical manner along the projection portion 71d. The helical depression portion 71e is formed along the helical projection portion 71d.

The heating unit 76a is a linear heater such as a heating wire or tape-shaped heater. The heating unit 76a is disposed along the depression portion 71e. Disposing the heating unit 76a in this manner allows uniformly heating the inside of the accordion pipe 71b from the outer peripheral surface of the accordion pipe 71b. As to an area of the accordion pipe 71b the heating unit 76a should be disposed in, various aspects, such as a position corresponding to the vicinity of the humidifying member 74, upstream or downstream from the humidifying member 74, and the entire outer peripheral surface of the accordion pipe 71b, are conceivable, and the present invention includes all of the aspects.

### <8. Respiratory assistance device 8>

### <8-1. Whole structure of respiratory assistance device 8>

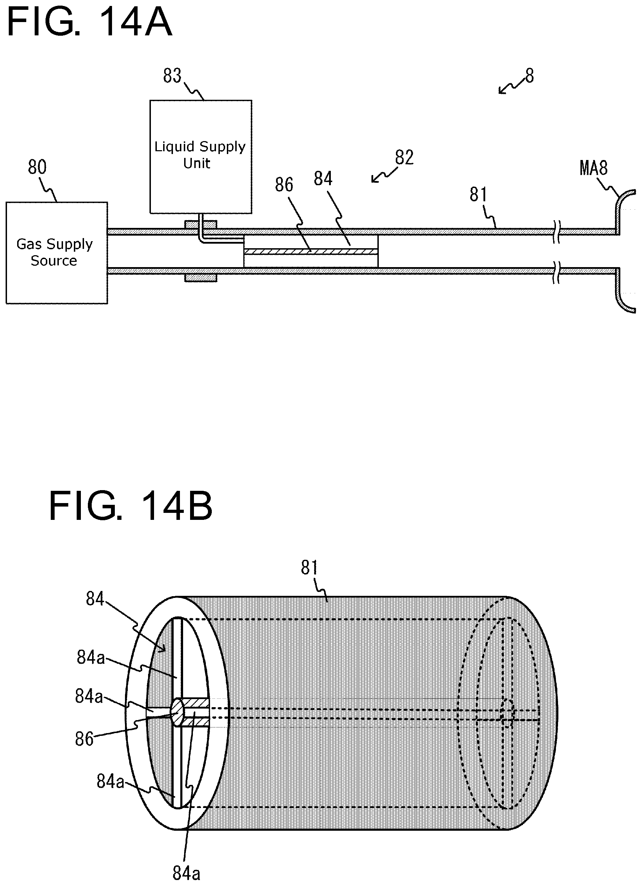

FIG. 14 is a drawing illustrating a respiratory assistance device 8 according to another embodiment of the present invention. FIG. 14A is a sectional view illustrating the whole structure of the respiratory assistance device 8. FIG. 14B is a drawing illustrating a humidifying member 84 in the respiratory assistance device 8.

As illustrated in FIG. 14A, the respiratory assistance device 8 includes a gas supply source 80, an inspiratory pipe 81, a humidifier 82, and the like.

Comparing the respiratory assistance device 1 with the respiratory assistance device 8, the gas supply source 80, the inspiratory pipe 81, and a liquid supply unit 83 are identical to those of the respiratory assistance device 1, and the description thereof has already been performed in the description about the respiratory assistance device 1 and hence is omitted. The difference between the respiratory assistance device 1 and the respiratory assistance device 8 includes the aspect of a humidifying member. This will be described below.

In the respiratory assistance device 8, a heating unit 86 is disposed so as to coincide with an axis of the inspiratory pipe 81. For example, as illustrated in FIG. 14B, in a humidifying member 84 of the respiratory assistance device 8, four plane members 84a are disposed so as to partition the inside of the inspiratory pipe 81 into four spaces. The plane members 84a are disposed so as to form a cross shape, as a whole, around the heating unit 86 as a center. As illustrated in FIG. 14B, the plane members 84a are disposed so as to extend along a length direction of the inspiratory pipe 81. Intake air passing through the inspiratory pipe 81 is supplied to a mask MA8 through the partitioned spaces. Note that the plane members 84a may be formed from any material out of all the types of humidifying members described above.

Disposing the heating unit 86 and the humidifying member 84 (the plane members 84a) as illustrated in FIG. 14B allows uniformly transmitting heat to the humidifying member 84, and uniformly heating the intake air inside the inspiratory pipe 81.

### <8-2. Modification example of humidifying member of respiratory assistance device 8>

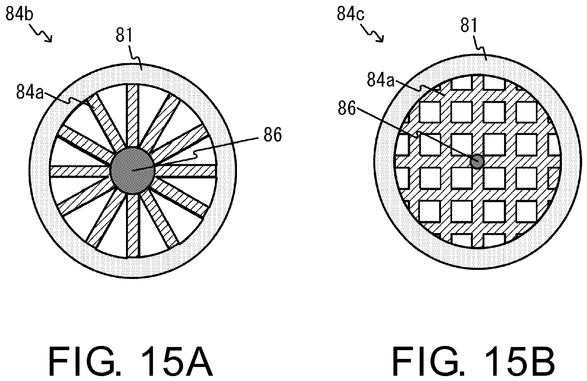

FIG. 15 is a drawing illustrating a modification example of the humidifying member of the respiratory assistance device 8. The humidifying member of the respiratory assistance device 8 is disposed so as to partition an interior space of the inspiratory pipe 81, and to pass intake air through the partitioned space. FIGS. 15A and 15B each illustrate an example of structure to achieve this.

FIG. 15A is a drawing illustrating a humidifying member 84b, i.e., a modification example of the humidifying member of the respiratory assistance device 8. While, as illustrated in FIG. 14B, the humidifying member 84 has the plane members 84a disposed in the cross shape, as illustrated in FIG. 15A, the humidifying member 84b has an increased number of plane members 84a (twelve plane members in FIG. 15A) extending radially from the center of the inspiratory pipe 81. The plane members 84a are disposed so as to extend along the length direction of the inspiratory pipe 81. The heating unit 86 is disposed at the center of the inspiratory pipe 81. Note that the heating unit 86 may be disposed in another position.

FIG. 15B is a drawing illustrating a humidifying member 84c, i.e., a modification example of the humidifying member of the respiratory assistance device 8. While both of the humidifying member 84 and the humidifying member 84b have the plurality of plane members disposed radially from the center of the inspiratory pipe 81, the humidifying member 84c has plane members 84a disposed into a lattice shape in the inspiratory pipe 81. The plane members 84a are disposed so as to extend along the length direction of the inspiratory pipe 81. The heating unit 86 is disposed at the center of the inspiratory pipe 81. Note that the heating unit 86 may be disposed in another position.

The present invention includes all of humidifiers and respiratory assistance devices that are obtained as combinations of elements of any of the above-described embodiments of the present invention.

The humidifiers and the respiratory assistance devices according to the present invention are not limited to the above-described embodiments, but, as a matter of course, can be variously modified without departing from the scope of the present invention.

### Reference Signs List

1, 2, 3, 4, 5, 6 respiratory assistance device
10, 20, 30, 40, 50, 60 gas supply source
11, 21, 31, 41, 51, 61 inspiratory pipe
11a inner peripheral surface
12, 22, 32, 42, 52, 62 humidifier
13, 23, 43 liquid supply unit
13a supply tube
13b, 24c, 24d, 24e leading unit
14, 44, 54, 63 container
15 injector
16, 25b three-way valve
17, 27, 37, 47, 57, 64, 64a, 64b, 67, 417, 517, 617, 717, 817, 917 humidifying member
17a, 17d, 17e, 17f water permeable fibers
18, 28, 38, 48, 67, 118, 228, 328, 418, 518, 618, 718
holder unit
18a, 18b, 28a, 28b, 38a, 38b holder member
18c, 18g, 118a, 228a, 328a, 418a, 518a, 618a, 718a
circular ring member
18d, 18h, 418b, 518b, 618b, 718b ring opening
18e, 28h hole
18f hollow interior
19, 19a, 19b, 19c, 26, 29, 39, 66, 68 heating unit
24 pump
24a supply tube
24b return tube
25 liquid replenishing unit
25a replenishing pipe
27a upper half member
27b lower half member
28a, 28b holder member
28c partition plate
45, 55 pressurization unit
56, 57 pressure measurement sensor
58 pressurization control unit
58a calculation unit
58b pressurization command unit
59 pressurizer
65 guide member
717a plane member
900 tank
S liquid circulation system

## Claims

1. A humidifier comprising:
an inspiratory pipe; and
a humidifying member configured to hold and vaporize a liquid, wherein
the humidifying member is configured in a manner selected from
a case where the humidifying member is disposed in a shape of a pipe along an inner periphery of the inspiratory pipe in a continuous or discontinuous manner in a circumferential direction, so that intake air passes through the pipe, and
a case where the humidifying member is disposed so as to partition an interior space of the inspiratory pipe, so that intake air passes through the partitioned space.

2. The humidifier according to claim 1, wherein the humidifying member has an aspect in which the humidifying member makes at least one round of the inner periphery of the inspiratory pipe.

3. The humidifier according to claim 1, wherein the humidifying member has an aspect in which a plurality of plane members are disposed radially.

4. The humidifier according to claim 1, wherein the humidifying member has an aspect in which a plurality of plane members are disposed into a lattice shape.

5. The humidifier according to any one of claims 1 to 4, wherein the humidifying member is formed from at least any one material out of woven cloth, nonwoven cloth, and a water-absorbing polymer.

6. The humidifier according to any one of claims 1 to 4, wherein the humidifying member is formed from a plurality of hollow fibers.

7. The humidifier according to claim 6, comprising:
a liquid supply unit configured to supply the liquid; and
a leading unit configured to lead the supplied liquid to end portion openings of the plurality of hollow fibers.

8. The humidifier according to claim 7, wherein
the liquid supply unit includes:
a pump configured to circulate the liquid in a liquid circulation system to supply the liquid to the plurality of hollow fibers;
a leading unit configured to lead the liquid so as to form a reciprocating tube together with the plurality of hollow fibers; and
a supply tube and a return tube that are connected to the leading unit corresponding to an inlet and an outlet of the reciprocating tube, respectively, to form the liquid circulation system together with the plurality of hollow fibers and the leading unit.

9. The humidifier according to claim 8, wherein:
the reciprocating tube is formed from
a first tube formed from a part of the plurality of hollow fibers, and
a second tube formed from a remaining part of the hollow fibers;
the leading unit is formed from
a first leading member configured to connect the supply tube to an end portion opening of the first tube,
a second leading member configured to connect the return tube to an end portion opening of the second tube, and
a third leading member configured to connect the other end portion opening of the first tube to the other end portion opening of the second tube; and
the liquid circulation system is formed from the supply tube and the return tube, the first tube, the second tube, the first leading member, the second leading member, and the third leading member.

10. The humidifier according to claim 8, wherein:
the reciprocating tube is formed by bending the plurality of hollow fibers into a reciprocating aspect;
the leading unit is formed from
a first leading member configured to connect the supply tube to one end portion openings of the plurality of hollow fibers, and
a second leading member configured to connect the return tube to other end portion openings of the plurality of hollow fibers; and
the liquid circulation system is formed from the supply tube and the return tube, the plurality of hollow fibers, the first leading member, and the second leading member.

11. The humidifier according to claim 7, wherein
the liquid supply unit includes
a container configured to contain the liquid; and
a feeding mechanism configured to gradually reduce a capacity of the container with time, to feed the liquid from the container and supply the liquid to the humidifying member.

12. The humidifier according to claim 7, wherein
the liquid supply unit includes
a container configured to contain the liquid, and
a pressurization unit configured to apply a pressure to an inside of the container.

13. The humidifier according to claim 12, wherein
the pressurization unit applies a pressure by, when the pressurization unit itself is retracted by an external force, feeding air held inside the pressurization unit itself to the inside of the container.

14. The humidifier according to any one of claims 12 and 13, wherein when a difference between a pressure of the inside of the container and a pressure of an inside of the inspiratory pipe is equal to or lower than a predetermined threshold value, the pressurization unit applies a pressure to the inside of the container.

15. The humidifier according to any one of claims 12 and 13, wherein when the pressure of the inside of the container is equal to or lower than a first threshold value, the pressurization unit applies a pressure to the inside of the container.

16. The humidifier according to any one of claims 12 and 13, wherein when a pressure of an inside of the inspiratory pipe is equal to or more than a second threshold value, the pressurization unit applies a pressure to the inside of the container.

17. The humidifier according to any one of claims 1 to 16, comprising a holder unit configured to hold the humidifying member so as to dispose the humidifying member in the shape of the pipe along the inner periphery of the inspiratory pipe in a continuous or discontinuous manner in the circumferential direction.

18. The humidifier according to claim 17, wherein the holder unit is a helical member.

19. The humidifier according to claim 17, wherein the holder unit is a ring continuation member in which ring-shaped members are continuously arranged and connected to each other in an axial direction of the inspiratory pipe.

20. The humidifier according to any one of claims 1 to 19, wherein a heating unit is provided outside or inside the inspiratory pipe, in order to heat the humidifying member.

21. The humidifier according to claim 20, wherein
when the humidifying member is disposed in the shape of the pipe along the inner periphery of the inspiratory pipe in a continuous or discontinuous manner in the circumferential direction,
the heating unit is provided with a heating unit configured to heat the inside of the inspiratory pipe from an outer peripheral side of the humidifying member, inside or outside the inspiratory pipe.

22. The humidifier according to any one of claims 20 and 21, wherein the heating unit is disposed helically on an outer periphery of the inspiratory pipe.

23. The humidifier according to claim 22, wherein
a projection portion is helically formed in the outer periphery of the inspiratory pipe, and
a depression portion is helically formed along the projection portion, and the heating unit is helically disposed in the depression portion.

24. The humidifier according to any one of claims 20 to 23, wherein the heating unit is disposed so as to heat the intake air inside the inspiratory pipe.

25. The humidifier according to any one of claims 20 to 24, wherein the heating unit is disposed so as to heat condensation water accumulated in the inspiratory pipe.

26. The humidifier according to any one of claims 1 to 4, comprising a container configured to contain the liquid, wherein
the humidifying member is disposed in the inspiratory pipe in a state where a part of the humidifying member is inserted in the container, to absorb the liquid from the container and vaporize the liquid.

27. The humidifier according to claim 26, wherein the humidifying member is formed from a water-absorbing material that can absorb the liquid in the container.

28. The humidifier according to any one of claims 26 and 27, wherein the humidifying member is formed from a water-absorbing material that can absorb the liquid in the container by capillarity.

29. The humidifier according to any one of claims 26 to 28, wherein the humidifying member is formed from a porous material.

30. The humidifier according to claim 29, wherein the porous material is formed from at least any one material out of woven cloth, nonwoven cloth, a hollow fiber, a porous water-absorbing polymer, and a porous synthetic resin.

31. The humidifier according to any one of claims 26 and 30, comprising a heating unit configured to heat the liquid in the container.

32. A respiratory assistance device comprising the humidifier according to any one of claims 1 to 31.
